# EUROPEAN PATENT APPLICATION

(11) **EP 4 094 582 A1**
(43) Date of publication of application: **30.11.2022**
(21) Application number: 21744816.6
(22) Date of filing: 19.01.2021
(51) Int. Cl.: A23C 9/152, A23L 33/125, A23L 33/135, C12N 1/20

(54) **COMPOSITION**

(30) Priority: 20.01.2020 JP 2020007101
(71) Applicant: Morinaga Milk Industry Co., Ltd., Minato-ku Tokyo 108-8384 (JP)
(72) Inventor: HARA Sakiko, Zama-shi, Kanagawa 252-8583 (JP); ODAMAKI Toshitaka, Zama-shi, Kanagawa 252-8583 (JP)
(74) Representative: Hoffmann Eitle
(86) International application number: PCT/JP2021/001603
(87) International publication number: WO 2021/149663

(57) **Abstract**

According to the invention, a composition which can promote the growth of a Bifidobacterial strain which can be a good bacterium is provided. The composition contains a human milk oligosaccharide mixture containing lacto-N-neotetraose, lacto-N-tetraose, 2'-fucosyllactose, 3'-sialyllactose, 6'-sialyllactose and *Bifidobacterium longum* subspecies *infantis.*

## Description

### Technical Field

The present invention relates to a composition.

The present application claims priority from patent application No. 2020-007101 filed on January 20, 2020 in Japan, and the contents thereof are incorporated here by reference.

### Background Art

In recent years, many studies on probiotics for the purpose of inhibiting onset of diseases or promoting health by actively taking bacteria which positively influence animals (also called "good bacteria" below) and regulating the enteric environment and on prebiotics such as components which help the growth of good bacteria have been conducted. While probiotics refer to bacteria which function beneficially in the intestines, prebiotics refer to substances which are selective nutrient sources for the probiotics and which promote the growth thereof. It is known that prebiotics have beneficial effects on human health such as the effect of promoting growth of lactic acid bacteria and *Bifidobacterium* spp., intestinal regulation effect and the effect of preventing or improving inflammatory bowel diseases.

Human milk oligosaccharides (also called "HMOs" below) are contained in breast milk in an amount of 10 to 20 g/L and are said to be a mixture of 130 kinds of oligosaccharide or more. The structures thereof are structures in which fucose and sialic acid are added to 13 kinds of core structure. In human milk, the proportion of fucosylated neutral saccharides is high, and 2'-fucosyllactose, lacto-N-fucopentaose I, lacto-N-difucohexaose I and lacto-N-tetraose are typical. The four kinds of oligosaccharide alone account for 1/3 to 1/4 of all the human milk oligosaccharides. Oligosaccharides including lacto-N-biose are called type I, and oligosaccharides including N-acetyllactosamine (LacNAc) are called type II. In human milk, the amounts of lacto-N-tetraose and lacto-N-fucopentaose I, which are type I, are higher than the amounts of lacto-N-neotetraose and lacto-N-fucopentaose III, which are type II. The coexistence of type I and type II and the priority of type I are distinctive features of human milk oligosaccharides which are different from milk oligosaccharides of other species (NPLs 1 and 2).

2'-FL and LNT, which are HMOs contained in breast milk, have the bifidobacteria-growing activity. 2'-FL and LNT are known to act as a growth factor for bifidobacteria by being selectively used by bifidobacteria and are important for the formation of intestinal microbiota in which bifidobacteria are dominant. HMOs can be synthesized and can be used industrially (PTL 1).

Bifidobacteria metabolize some HMOs using the GNB/LNB pathway that is specific to bifidobacteria. Although many of the species commonly found in the human intestinal tract have the pathway, most of the bifidobacteria commonly found in the intestinal tracts of animals other than human and insects do not have the pathway. Typical examples of the bacterial species which can grow using HMOs as the sole carbon source are four bacterial species of *Bifidobacterium longum* subspecies *longum, Bifidobacterium longum* subspecies *infantis, Bifidobacterium breve* and *Bifidobacterium bifidum,* which are bifidobacteria commonly found in infants. In particular, two bacterial species thereof, namely *Bifidobacterium longum* subspecies *infantis* and *Bifidobacterium bifidum,* have been reported to use various HMOs (NPL 3).

### Citation List

### Patent Literature

PTL 1: WO2014/086373

### Non Patent Literature

NPL 1: Japanese Journal of Lactic Acid Bacteria Vol. 22, No. 1, pp15-25 2011
NPL 2: Milk Science Vol. 56, No.4, pp155-176 2008
NPL 3: Milk Science Vol. 61, No.2, pp115-124 2012

### Summary of Invention

### Technical Problem

To actively take a good bacterium and regulate the enteric environment, it is important to take the good bacterium in an environment in which the growth of the good bacterium is promoted.

A problem of the invention is to provide a composition which can promote the growth of a Bifidobacterial strain which can be a good bacterium.

### Solution to Problem

The present inventors have examined the growth of a Bifidobacterial strain in an environment containing HMOs to solve the problem and have found that the degree of the growth of the Bifidobacterial strain differed with the kinds and the combination of HMOs. As a result of further extensive studies on the kinds and the combination of HMOs suitable for the growth of the Bifidobacterial strain, the inventors have succeeded in constructing a composition of an HMO mixture which is suitable for the growth of the Bifidobacterial strain and thus have completed the invention.

That is, in the invention, first, a composition containing
a human milk oligosaccharide mixture containing
lacto-N-neotetraose,
lacto-N-tetraose,
2'-fucosyllactose,
3'-sialyllactose,
6'-sialyllactose and
*Bifidobacterium longum* subspecies *infantis* is provided.

As the *Bifidobacterium longum* subspecies *infantis* used for the composition according to the invention, *Bifidobacterium longum* subspecies *infantis* NITE BP-02623 can be used.

The human milk oligosaccharide mixture used for the composition according to the invention can contain
the lacto-N-neotetraose in an amount of 2 to 10 mass%,
the lacto-N-tetraose in an amount of 10 to 20 mass%,
the 2'-fucosyllactose in an amount of 55 to 75 mass%,
the 3'-sialyllactose in an amount of 1 to 10 mass% and
the 6'-sialyllactose in an amount of 5 to 15 mass%.

The human milk oligosaccharides used for the composition according to the invention can further include difucosyllactose or 3'-fucosyllactose.

In this case, the human milk oligosaccharide mixture used for the composition according to the invention can contain
the difucosyllactose or the 3'-fucosyllactose in an amount of 1 to 10 mass%.

The composition according to the invention can further contain lactose.

In the invention, a composition containing
lacto-N-tetraose and
*Bifidobacterium longum* subspecies *infantis* is also provided.

The compositions according to the invention can be used as a food or a drink composition, a nutritional composition or a formula.

### Advantageous Effects of Invention

When the compositions according to the invention are used, the growth of a Bifidobacterial strain which can be a good bacterium can be promoted. The effect described here is not necessarily limited and may also be any of the effects described in the invention.

### Brief Description of Drawings

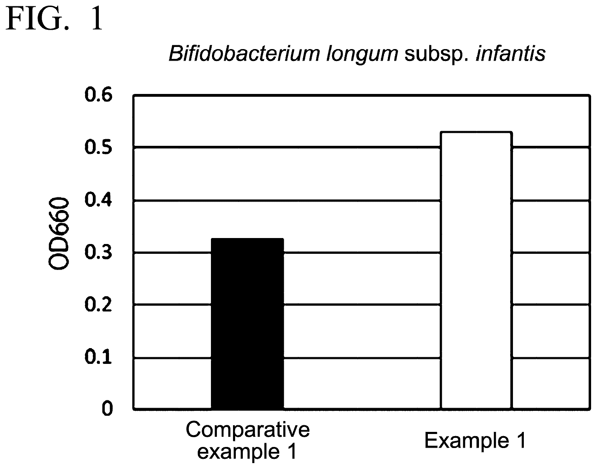

[Fig. 1] Fig. 1 is a graph showing the growth activities of *Bifidobacterium longum* subspecies *infantis* at the saccharide ratios of Example 1 and Comparative Example 1.

### Description of Embodiments

Embodiments for carrying out the invention are explained below. In this regard, the embodiments explained below show examples of typical embodiments of the present disclosure, and the scope of the invention is not construed as being narrower due to the embodiments. In the present specification, in a numerical range expressed with "a lower limit to an upper limit", the upper limit may be "the value or less" or "less than the value", and the lower limit may be "the value or more" or "more than the value". Moreover, the percentages in the present specifications are based on mass unless otherwise specified.

### 1. Compositions

A composition according to the invention contains at least a human milk oligosaccharide mixture and *Bifidobacterium longum* subspecies *infantis.* Moreover, a composition according to the invention contains at least lacto-N-tetraose and *Bifidobacterium longum* subspecies *infantis.* The compositions according to the invention can further contain lactose or the like.

### (1) Human Milk Oligosaccharide Mixture

The human milk oligosaccharide mixture used in the invention contains the HMOs below.
a. lacto-N-neotetraose (also called "LNnT" below)
b. lacto-N-tetraose (also called "LNT" below)
c. 2'-fucosyllactose (also called "2'-FL" below)
d. 3'-sialyllactose (also called "3'-SL" below)
e. 6'-sialyllactose (also called "6'-SL" below)

The human milk oligosaccharide mixture used in the invention may contain the HMOs below.

f. difucosyllactose (also called "DFL" below) or 3'-fucosyllactose (also called "3'-FL" below)

The HMO contents of the HMO mixture can be appropriately set depending on the purpose but are preferably set in the respective ranges below.
a. LNnT: 2 to 10 mass%
b. LNT: 10 to 20 mass%
c. 2'-FL: 55 to 75 mass%
d. 3'-SL: 1 to 10 mass%
e. 6'-SL: 5 to 15 mass%
f. DFL or 3'-FL: 1 to 10 mass%

The HMO mixture used in the invention can also contain one, two or more kinds of generally known HMOs in addition to the HMOs. Examples of the HMOs include 3-difucosyllactose, 3-fucosyl-3'-sialyllactose, lacto-N-fucopentaose I, lacto-N-fucopentaose II, lacto-N-fucopentaose III, lacto-N-fucopentaose V, lacto-N-difucosylhexaose I, lacto-N-difucosylhexaose II, lacto-N-sialylpentaose, LSTa, LSTb and LSTc.

The HMOs contained in the HMO mixture used in the invention may be commercial products, may be prepared from milk or may be obtained by a known method such as organic synthesis and enzymatic treatment. Milk (for example, breast milk, milk formula, cow's milk or a dairy product) containing the HMOs may also be used. Moreover, a composition obtained by blending the HMOs with milk (for example, cow's milk, milk formula or infant milk) (that is, milk containing the HMOs) may also be used.

In this regard, the LNT used in the invention does not have to be used as the HMO mixture explained above, and the composition according to the invention may be a composition containing LNT and the bacterium belonging to *Bifidobacterium longum* subspecies *infantis* described below.

### (2) Bacterium Belonging to Bifidobacterium longum subspecies infantis

As the bacterium belonging to *Bifidobacterium longum* subspecies *infantis* ("*Bifidobacterium infantis*" is reclassified as "*Bifidobacterium longum* subspecies *infantis*" and is synonymous) which can be used in the invention, one, two or more kinds of known or unknown bacteria belonging to *Bifidobacterium longum* subspecies *infantis* can be freely selected and used as long as the effects of the invention are not impaired. Specifically, examples thereof include *Bifidobacterium longum* subspecies *infantis* (NITE BP-02623), *Bifidobacterium longum* subspecies *infantis* (ATCC15697T) and the like.

*Bifidobacterium longum* subspecies *infantis* (NITE BP-02623) was deposited for an international deposit under the Budapest Treaty on January 26, 2018 to NITE Patent Microorganisms Depositary, National Institute of Technology and Evaluation (Room 122, 2-5-8 Kazusakamatari, Kisarazushi, Chiba 292-0818).

*Bifidobacterium longum* subspecies *infantis* (ATCC15697T) can be acquired from ATCC, American Type Culture Collection (10801 University Boulevard, Manassas, Virginia 20110-2209, U.S.A.).

The bacterial strains specified by the bacterial strain names exemplified above are not limited to the strains deposited or registered at the certain organizations under the bacterial strain names themselves (also called "deposited strains" below for the convenience of explanation) but include substantially equivalent strains thereof (also called "derived strains" or "induced strains" below). That is, for example, "*Bifidobacterium longum* subspecies *infantis* (NITE BP-02623)" is not limited to the strain deposited to the depositary with the deposition number of *Bifidobacterium longum* subspecies *infantis* (NITE BP-02623) itself but includes substantially equivalent strains thereof.

Examples of the substantially equivalent strains of the deposited strains may be strains derived from the deposited strains as parent strains. The derived strains include strains bred from the deposited strains or strains naturally generated from the deposited strains.

The derived strains are the following strains.
(1) A bacterial strain which is determined to be the identical bacterial strain by the Randomly Amplified Polymorphic DNA method or the Pulsed-field gel electrophoresis method (described in Probiotics in food/Health and nutritional properties and guidelines for evaluation 85 Page43).
(2) A bacterial strain which has genes derived from the deposited strain only but does not have any exogenous gene and which has a DNA identity of 95% or more.
(3) A strain bred from the bacterial strain or a strain which includes modification by genetic engineering, a mutation or a spontaneous mutation and which has the identical characters.

The growth of such a bacterium belonging to *Bifidobacterium longum* subspecies *infantis* is promoted in the presence of the HMO mixture described above. That is, when the HMO mixture described above is used, such a bacterium belonging to *Bifidobacterium longum* subspecies *infantis* can be grown easily in the intestines.

Bacteria belonging to *Bifidobacterium longum* subspecies *infantis* have been reported to have various physiological functions, and the functions have been reported to be due to the growth in the intestines and the produced substances (for example, acetic acid). Accordingly, the bacterium belonging to *Bifidobacterium longum* subspecies *infantis* of the invention can also be expected to be highly safe and show the generally known effects of the bacteria belonging to *Bifidobacterium longum* subspecies *infantis* in a wide age group. Therefore, the compositions of the invention can be used for a wide range of applications (for a food or a drink, for a functional food, for a pharmaceutical product, for feed or the like). Moreover, the bacterium belonging to *Bifidobacterium longum* subspecies *infantis* can be expected to have a probiotic effect, and thus the compositions of the invention can also be used for the purpose of promoting the health, improving the diet, improving the enteric environment, preventing·treating an intestinal infection or the like.

The bacterium belonging to *Bifidobacterium longum* subspecies *infantis* used in the invention can be grown, for example, by culturing a bacterial strain thereof.

The culture method is not particularly limited as long as the bacterium belonging to *Bifidobacterium longum* subspecies *infantis* can be grown, and a method generally used for culturing a bacterium belonging to *Bifidobacterium longum* subspecies *infantis* can be used with appropriate modification when necessary. For example, the culture temperature may be 30 to 50°C and is preferably 35 to 45°C. The bacterium is preferably cultured under an anaerobic condition and can be cultured, for example, while sending an anaerobic gas such as carbon dioxide gas. Furthermore, the bacterium may be cultured under a microaerophilic condition such as static liquid culture.

The medium for culturing the bacterium belonging to *Bifidobacterium longum* subspecies *infantis* used in the invention for the growth is not particularly limited, and a medium which is generally used for culturing the bacterium belonging to *Bifidobacterium longum* subspecies *infantis* used in the invention can be used with appropriate modification when necessary. That is, as a carbon source other than the HMOs, for example, saccharides such as galactose, glucose, fructose, mannose, cellobiose, maltose, lactose, sucrose, trehalose, starch hydrolysate and molasses can be used depending on the assimilation properties. As a nitrogen source, for example, ammonia and ammonium salts or nitrates such as ammonium sulfate, ammonium chloride and ammonium nitrate can be used. Moreover, as an inorganic salt, for example, sodium chloride, potassium chloride, potassium sulfate, magnesium sulfate, calcium chloride, calcium nitrate, manganese chloride, ferrous sulfate or the like can be used. Furthermore, organic components such as peptone, soybean powder, a defatted soybean cake, meat extract and yeast extract may also be used. In addition, as a modified medium, for example, MRS medium can be used.

### (3) Others

The compositions according to the invention can also contain any component as long as the effects of the invention are not impaired. Examples thereof which can be appropriately used include: fatty components such as coconut oil, soybean oil, monoglyceride and diglyceride; carbohydrates such as glucose, lactose and starch hydrolysate; protein such as soy protein and milk protein; and vitamins and minerals (calcium, phosphorus, potassium, sodium, chlorides, magnesium, manganese, iron, copper, zinc, selenium, iodine, vitamins A, E, D, C and B and complexes thereof).

### 2. Applications of Compositions According to Invention

By administering a composition of the invention to a human, the effect of promoting the growth of a probiotic affecting good bacteria can be expected. That is, when the probiotic grows in the intestines, the enteric microbiota is improved. When the enteric microbiota is improved, a disease caused due to the enteric microbiota is improved or prevented, and treatment thereof becomes possible.

The disease caused due to the enteric microbiota is inflammatory colitis, ulcerative colitis, irritable colitis, colon cancer, diabetes, arteriosclerosis or the like.

Moreover, by administering a composition of the invention, immune function is enhanced. When immune function is enhanced, an allergy such as pollinosis, asthma and atopic dermatitis can be prevented, improved or treated.

The compositions according to the invention can be preferably used for a food or a drink, an infant formula, a pharmaceutical product, a quasi-drug, feed or the like.

The application of the embodiment may be a use for therapeutic purpose or may be a use for non-therapeutic purpose.

The "non-therapeutic purpose" is a concept which does not include medical practice, namely treatment of a human body by therapy. Examples thereof include health promotion and beauty treatment.

The "improvement" means: improvement of a disease, a symptom or the condition; prevention or delay of deterioration of a disease, a symptom or the condition; or reversal, prevention or delay of progress of a disease or a symptom.

The "prevention" means prevention or delay of the onset of a disease or a symptom in the subject of the application or reduction of the risk of a disease or a symptom of the subject of the application.

In the compositions according to the invention, the HMO mixture or LNT content can be freely set as long as the effects of the invention are not impaired. In the invention, in particular, the HMO mixture or LNT content of the composition can be set at 0.05% to 20.0% relative to the final composition.

Moreover, in the invention, the daily dosage of the HMO mixture or LNT can also be freely set as long as the effects of the invention are not impaired. In the invention, in particular, the dosage is preferably set at an equivalent value to the HMO concentration contained in general breast milk. Specifically, the dosage can be set at, for example, 200 mg to 20 g/day, preferably 300 mg to 15 g/day, more preferably 400 mg to 10 g/day, further preferably 500 mg to 10 g/day, most preferably 1 g to 10 g/day.

Regarding the compositions according to the invention, the bacterium belonging to *Bifidobacterium longum* subspecies *infantis* is preferably administered at, relative to the final compositions, about 1×10³ to about 1×10¹² CFU/g or mL, but a higher value is also acceptable. When the compositions are administered to an adult human (namely, a human over 16 years of age), the bacterium belonging to *Bifidobacterium longum* subspecies *infantis* is administered preferably at least at 1×10⁶ CFU/g or mL or more, more preferably at least at 1×10⁸ CFU/g or mL or more. When the compositions are administered to a child (namely, a human under 16 years of age), the bacterium belonging to *Bifidobacterium longum* subspecies *infantis* is administered in the range of preferably about 1×10⁶ to about 1×10¹¹ CFU/g or mL, more preferably about 1×10⁶ to about 1×10⁹ CFU/g or mL. In the invention, CFU means the colony forming unit and indicates the colony forming unit. When the bacterium is a dead bacterium, CFU can be replaced with cells.

The amount of the bacterium belonging to *Bifidobacterium longum* subspecies *infantis* used is not particularly restricted due to the high safety but is preferably, for example, 1×10⁶ to 1×10¹² CFU/kg body weight/day, more preferably 1×10⁷ to 1×10¹¹ CFU/kg body weight/day, further preferably 1×10⁸ to 1×10¹⁰ CFU/kg body weight/day. Alternatively, the used amount (dosage) per individual (body weight) is preferably 10⁷ to 10¹⁴ CFU/day, more preferably 10⁸ to 10¹³ CFU/day, and further preferably 10⁹ to 10¹² CFU/day.

The subject of the administration of the invention is preferably an infant or a small child.

The term infant or small child includes an infant and a small child, further specifically includes a baby, a small child and a newborn and further specifically includes a baby, a small child, a newborn, an immature baby, a premature baby and a low birthweight baby. In the invention, the species of the infant or the small child includes human unless otherwise specified. The infant refers to a child in the infancy. The infancy means the period in which the subject takes milk such as breast milk as the major source of nutrient, and the period before the age of 1 year is generally the infancy in the case of human. The small child generally refers to a child in the period before entering school. The newborn refers to a child in the neonatal period. The neonatal period means the period shortly after birth, and the period within four weeks after birth is generally the neonatal period in the case of human.

In the invention, a prebiotic may also be used in combination in addition to the HMOs and the bacterium belonging to *Bifidobacterium longum* subspecies *infantis* described above. The prebiotic is a resistant food component which has an advantageous effect on the host and improves the health of the host by selectively changing the growth and the activities of a specific bacterium in the large intestine.

The prebiotic is not particularly restricted as long as the effects of the invention can be exerted more when the prebiotic is taken with the bacterium or a culture thereof, but examples thereof include lactulose, raffinose, a galactooligosaccharide, a fructooligosaccharide, a soybean oligosaccharide, lactosucrose, a xylooligosaccharide, an isomaloligosaccharide, a coffee bean mannooligosaccharide, gluconic acid, polydextrose and inulin. Lactulose, raffinose and a galactooligosaccharide are preferable, and lactulose, raffinose and a galactooligosaccharide are more preferable.

### <Nutritional Composition>

The compositions according to the invention can be used as a nutritional composition. In the invention, the "nutritional composition" refers to a food or a drink which is orally or intravenously taken and provides nutrients to the subject of the inoculation. The kind of the nutritional composition which can be used in the invention is not particularly limited but is preferably a formula, a liquid food or the like, more preferably a formula. The subject of the intake may be an infant, a small child, a child or an adult but is preferably an infant or a small child.

The infant formulas includes a powdered formula and a liquid formula.

The powdered formula is defined by the Ministerial Ordinance Concerning Compositional Standards, etc. for Milk and Milk Products as "those obtained by processing raw milk, cow's milk, certified milk or a food produced therefrom as a raw material or using such a material as a major raw material, adding nutrients necessary for infants and small children and processing into powder".

The liquid formula is defined by the Ministerial Ordinance as "those obtained by processing raw milk, cow's milk, certified milk or a food produced therefrom as a raw material or using such a material as a major raw material, adding nutrients necessary for infants and small children and processing into liquid".

The formulas also includes those which contain a nutrient component such as proteins, oils and fats, carbohydrates, minerals and vitamins and which are processed into powder or liquid.

The formulas further includes "a powdered infant formula", "a liquid infant formula" and "a powdered formula for pregnant and parturient women and nursing mothers" of the food for special dietary uses provided by the Health Promotion Act and also includes the forms of a powdered infant formula for infants and small children for infants of the age of 0 to 12 months, follow-up milk for infants and younger children of the age of 6 to 9 months or older (until the age of 3 years), a powdered infant formula for low birthweight infants for newborns with a body weight at birth less than 2500 g (low birthweight infants), formula for treatment including formula for allergies used for the treatment of infants and small children with sickness such as milk allergy and lactose intolerance, an infant formula for lactose intolerance and an infant formula for inborn errors of metabolism, a powdered infant formula for small children, an infant formula for school children, an adult nutritional formula , a formula for the aged, baby food, food for small children, an enhancer agent for breast milk or an infant formula, nutritional powder for adults, nutritional powder for the aged and the like. The composition can also be applied to food with health claims or food for the ill. The system for food with health claims has been established not only for general foods but also for foods in the form of tablets, capsules and the like, in view of the trends inside and outside of Japan and also considering the consistency with the existing system for foods for specified health uses. The system includes two types, namely foods for specified health uses (individual approval system) and foods with nutrient function claims (standard regulation system).

In the invention, the definitions of the nutritional compositions, the formula, the foods and the like include those defined by countries, international institutions, cooperative institutions, cooperative organizations, institutions belonging thereto, organizations, groups, associations and the like.

### <Food or Drink>

The compositions according to the invention can also be prepared by adding to a known food or drink. A new food or drink can also be produced by mixing a composition in a raw material of a food or a drink.

The food or the drink containing a composition according to the invention is not limited regarding the form such as liquid, paste, solid and powder, and examples thereof include, in addition to tablet candies, liquid foods and the like, wheat products, instant foods, processed agricultural products, processed fishery products, processed livestock products, milk/dairy products, oils and fats, basic condiments, compound flavor enhancers/foods, frozen foods, confectioneries, beverages and other commercial products.

Examples of the wheat products include breads, macaroni, spaghetti, noodles, cake mixes, frying flours and bread crumbs.

Examples of the instant foods include instant noodles, cup noodles, retort-pouched/prepared foods, prepared canned foods, microwave foods, instant soups/stews, instant miso soups/clear Japanese soups, canned soups, freeze-dried foods and other instant foods.

Examples of the processed agricultural products include canned agricultural products, canned fruits, jams/marmalades, pickles, cooked beans, dried agricultural products and cereals (processed grains).

Examples of the processed fishery products include canned fishery products, fish hams/sausages, fishery paste products, fishery delicacies and *Tsukudani* (foods boiled down in sweetened soy sauce).

Examples of the processed livestock products include canned livestock products/pastes and livestock hams/sausages.

Examples of the milk/dairy products include fermented milk, processed milk, milk beverages, yogurts, lactic acid bacteria beverages, cheeses, ice creams, formulas, creams and other dairy products.

Examples of the oils and fats include butter, margarine and vegetable oils.

Examples of the basic condiments include soy sauce, soybean paste, sauces, processed tomato condiments, *Mirin* (sweet sake for seasoning) and vinegars.

The compound flavor enhancers/foods include cooking mixes, curry roux, sauces, dressings, noodle broths, spices and other compound flavor enhancers.

Examples of the frozen foods include frozen food materials, semi-cooked frozen foods and cooked frozen foods.

Examples of the confectioneries include caramels, candies, chewing gums, chocolates, cookies, biscuits, cakes, pies, snacks, crackers, Japanese-style confectioneries, rice confectioneries, bean confectioneries, desserts and other confectioneries.

Examples of the beverages include carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols and other luxury beverages.

Examples of the other commercial foods include baby foods, *Furikake* (dry Japanese seasonings) and seasonings for *Chazuke* (boiled rice with hot tea).

### [Food or Drink with Functional Label]

The food, the drink or the like defined in the invention can also be provided or sold as a food or a drink with a label with a specific use (especially a health use) or a function.

The "labeling" act includes all the acts for informing a consumer of the use, and all the expressions which can remind of/cause to guess the use are the "labeling" acts of the invention, regardless of the purposes of labeling, the contents of labeling, the objects to be labeled, the media and the like.

The "label" is preferably with an expression which allows a consumer to directly recognize the use. Specific examples include an act of transferring an article in which the use is described on a product regarding the food or the drink or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (internet or the like) and another act.

The content of the label is preferably a label approved by the administration or the like (for example, a label approved based on a system provided by the administration and provided in the form based on the approval). It is preferable to label with such a content on packaging, a container, a catalogue, a brochure, an advertisement material in a sales site such as POP, other documents or the like.

The "labels" also include labels with health foods, functional foods, foods for the ill, enteral nutrition products, food for special dietary uses, food with health claims, foods for specified health uses, foods with function claims, foods with nutrient function claims, quasi-drugs and the like. In particular, the labels are labels approved by the Consumer Affairs Agency, such as labels approved by the systems for foods for specified health uses, the systems for foods with function claims and similar systems thereof. More specific examples include a label with foods for specified health uses, a label with qualified foods for specified health uses, a label with foods with function claims, a label indicating influence on the structure and the function of a body, a label with reduction of disease risk and the like. Typical examples thereof include labels with food for specified health uses (especially labels with health uses) provided by the Regulations for Enforcement of the Health Promotion Act (Order of the Ministry of Health, Labor and Welfare of Japan, No. 86, April 30, 2003), labels with foods with function claims provided by the Food Labeling Act (Act No. 70 of 2013) and similar labels.

By administering a composition according to the invention, the bacterium belonging to *Bifidobacterium longum* subspecies *infantis* grows in the intestines of the subject of the administration. When the probiotic grows in the intestines, the immunocompetence improves, and the enteric flora improves. In particular, in infants and small children, by administering a composition according to the invention, bifidobacteria in the intestines increase effectively, and the enteric environment is regulated. By taking in bad bacteria or opportunistic bacteria together with the Bifidobacterial strain, immunocompetence can be acquired, and the resulting enteric environment such as the enteric bacterial flora is kept as it is in adults.

Regarding the method for producing the formula, one, two or more kinds of known production methods can be freely combined and used as long as the effects of the invention are not impaired. Specifically, for example, the modified milk can be produced by mixing a composition according to the invention and additives in raw material milk and through steps such as homogenization, sterilization, concentration, drying, granulation and sieving.

The formula of the invention can be specifically produced, for example, by the following method.

That is, the invention provides a method for producing a formula including mixing the HMO mixture or LNT and a bacterial powder containing a bacterium belonging to *Bifidobacterium longum* subspecies *infantis* and thus obtaining a formula.

Specifically, for example, the invention provides a method for producing a formula including the step A and the step B below:
step A: a step of culturing a bacterium belonging to *Bifidobacterium longum* subspecies *infantis* in a medium containing any component and thus obtaining a culture; and
step B: a step of mixing the culture and the HMO mixture or LNT and obtaining a formula.

The food composition of the invention may be specifically, for example, a supplement for infants and small children. Examples of the supplement for infants and small children can be produced, for example, by the following method.

That is, the invention provides, for example, a method for producing a supplement including the step C to the step E below:
step C: a step of culturing a bacterium belonging to *Bifidobacterium longum* subspecies *infantis* in a medium containing any component and thus obtaining a culture;
step D: a step of mixing the culture, the HMO mixture or LNT and an excipient and thus obtaining a mixture; and
step E: a step of formulating the mixture by tableting or the like.

In this regard, the medium used for the step A and the step C above can contain the HMO mixture or LNT.

In all the production methods described above, a component other than the components cited in the steps may be appropriately used in combination.

The method for adding a composition according to the invention is not particularly limited as long as the effects of the invention are not impaired, but for example, the composition according to the invention can be added in a liquid state during mixing as described above or can be formed into powder and then added for powder mixing and production.

The HMO mixture or LNT content of the formula according to the invention can be freely set as long as the effects of the invention are not impaired. In the invention, in particular, the HMO mixture or LNT content of the modified milk can be set at 0.05% to 20.0% relative to the final composition of the formula.

The amount of the bacterium belonging to *Bifidobacterium longum* subspecies *infantis* in the formula according to the invention can be freely set as long as the effects of the invention are not impaired. In the invention, in particular, the amount of the bacterium belonging to *Bifidobacterium longum* subspecies *infantis* in the formula can be set at 1×10³ to 1×10¹² CFU/mL or g relative to the final composition of the formula.

### <Pharmaceutical Product and Quasi-Drug>

A composition according to the invention may be added to a pharmaceutical product or a quasi-drug (also called "a pharmaceutical product or the like" below) which is known. A new pharmaceutical product or the like can be produced by mixing the composition according to the invention in a raw material of a pharmaceutical product or the like.

When the composition according to the invention is contained in a pharmaceutical product or the like, the pharmaceutical product or the like can be appropriately formulated into a desired dosage form depending on the administration method such as oral administration or parenteral administration. The dosage form is not particularly limited, but in the case of oral administration, for example, the pharmaceutical product or the like can be formulated into a solid preparation such as powder, granules, tablets, troches and capsules, a liquid preparation such as a solution, a syrup, a suspension and an emulsion or the like. In the case of parenteral administration, for example, the pharmaceutical product or the like can be formulated into a suppository, spray, inhalant, ointment, a plaster or an injection. In the invention, formulation into a dosage form for oral administration is preferable.

The formulation can be appropriately conducted by a known method depending on the dosage form.

The formulation may also be conducted, for example, by appropriately blending a carrier for formulation. Moreover, in addition to the peptide of the invention, a component which is generally used for formulation, such as excipients, pH-adjusting agents, colorants and corrigents, can be used. A component having the effect of preventing, improving and/or treating a disease or a symptom which is known or will be found in the future can also be appropriately used in combination depending on the purpose.

As the carrier for formulation, an organic or inorganic carrier can be used depending on the dosage form. Examples of the carrier for a solid preparation include excipients, binders, disintegrating agents, lubricants, stabilizers and flavoring agents.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; and sulfate derivatives such as calcium sulfate.

Examples of the binders include, in addition to the excipients, gelatin, polyvinylpyrrolidone and macrogol.

Examples of the disintegrating agents include, in addition to the excipients, chemically modified starch or cellulose derivatives such as croscarmellose sodium, sodium carboxymethyl starch and cross-linked polyvinylpyrrolidone.

Examples of the lubricants include: talc; stearic acid; metal stearates such as calcium stearate and magnesium stearate; colloidal silica; waxes such as peegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; and starch derivatives.

Examples of the stabilizers include: paraoxybenzoate esters such as methylparaben and propylparaben; alcohols such as chlorobutanol, benzyl alcohol and phenylethyl alcohol; benzalkonium chloride; acetic anhydride; and boric acid.

Examples of the flavoring agents include sweeteners, acidulants and aromas.

In this regard, examples of the carrier used in the case of a liquid preparation for oral administration include solvents such as water and flavoring agents.

### <Feed>

The compositions according to the invention may be added to known feed. Feed can be produced by mixing a composition in a raw material of feed.

When a composition according to the invention is contained in feed, examples of the raw material of the feed include: grains such as corn, wheat, barley and rye; bran such as oat bran, wheat bran, rice bran and defatted rice bran; food manufacturer's by-products such as corn gluten meal and corn jam meal; animal feed such as defatted powder milk, whey, fish powder and bone meal; yeasts such as brewer's yeast; mineral feed such as calcium phosphate and calcium carbonate; oils and fats; amino acids; and saccharides. Examples of the form of the feed include feed for pets such as pet food, livestock feed and fish farming feed.

### Examples

The invention is explained in further detail below based on an Example. In this regard, the Example explained below shows an example of typical Examples of the invention, and the scope of the invention is not construed as being narrower due to the Example.

As an example of the bacterium belonging to *Bifidobacterium longum* subspecies *infantis, Bifidobacterium longum* subspecies *infantis* NITE BP-02623 possessed by Morinaga Milk Industry Co., Ltd. was seeded at 1 v/v% as a bacterial solution to 200 µL of a medium after 16 hours of degassing and cultured under an anaerobic condition at 37°C. As the media, MRS (de Man-Rogosa-Sharpe) liquid media (composition: Table 2) in which the saccharide source was changed to the human milk oligosaccharides (manufactured by Jennewein Biotechnologie GmbH) shown in Table 1 were each produced. The turbidities (OD660) after 16 hours of culturing were measured, and the growth of *Bifidobacterium longum* subspecies *infantis* was compared between the saccharide ratios of Example 1 and Comparative Example 1.

### [Table 1]

**Table 1. Saccharide Source Compositions**

| | | 2'FL | LNnT | LNT | 3'SL | 6'SL |
|---|---|---|---|---|---|---|
| Example 1 | Used Amount (g/L) | 5.12 | 0.4 | 1.12 | 0.4 | 0.72 |
| | Amount in HMOs (mass%) | 66.0 | 5.2 | 14.4 | 5.2 | 9.3 |
| Comparative Example 1 | Used Amount (g/L) | 1 | - | - | 2 | 1 |
| | Amount in HMOs (mass%) | 25.0 | - | - | 50.0 | 25.0 |

### [Table 2]

**Table 2. MRS Composition**

| | |
|---|---|
| BBL Polypeptone (manufactured by BD Bioscience) | 10 g |
| Bacto Beef Extract (Desicated, manufactured by BD Bioscience) | 10 g |
| Bacto Yeast Extract (manufactured by BD Bioscience) | 5 g |
| Polyoxyethylene sorbitan monooleate (manufactured by Nacalai) | 1 g |
| Dipotassium hydrogenphosphate (manufactured by Kokusan Chemical Co., Ltd.) | 2 g |
| Sodium acetate (manufactured by Wako Pure Chemical Industries, Ltd.) | 5 g |
| Diammonium hydrogen citrate (manufactured by Wako Pure Chemical Industries, Ltd.) | 2 g |
| Magnesium sulfate heptahydrate (manufactured by Wako Pure Chemical Industries, Ltd.) | 0.2 g |
| Manganese(II) sulfate pentahydrate (manufactured by Wako Pure Chemical Industries, Ltd.) | 0.05 g |
| Distilled water | 800 mL |
| SW (added later) | 200 mL |
| Total | 1000 mL |
| pH | 6.5 |

The results are shown in Fig. 1. As shown in the graph of Fig. 1, a high growth activity was shown in the case of the saccharide ratio of the Example as compared to the case of the saccharide ratio of Comparative Example 1. From the results, it was demonstrated that the growth of *Bifidobacterium longum* subspecies *infantis* is promoted when an HMO mixture containing LNnT, LNT, 2'-FL, 3'-SL and 6'-SL is used.

### [Production Examples]

Production Examples of a composition, a pharmaceutical composition, a fermented milk and a formula are shown below.

### <Production Example 1>

*Bifidobacterium longum* subspecies *infantis* is added to 3 mL of MRS medium and anaerobically cultured at 37°C for 24 hours, and then the culture solution is concentrated and lyophilized to obtain lyophilized powder. The obtained lyophilized powder is uniformly mixed with LNnT, LNT, 2'-FL, 3'-SL and 6'-SL, and a composition is thus obtained.

### <Production Example 2>

*Bifidobacterium longum* subspecies *infantis* is added to 3 mL of MRS medium and anaerobically cultured at 37°C for 24 hours, and then the culture solution is concentrated and lyophilized to obtain lyophilized powder. Next, the obtained lyophilized powder, LNnT, LNT, 2'-FL, 3'-SL, 6'-SL and crystalline cellulose are introduced into an agitation granulator and mixed. Then, purified water is added for granulation, and the granules are dried to obtain granules (a pharmaceutical composition).

### <Production Example 3>

A production method of a fermented milk containing *Bifidobacterium longum* subspecies *infantis* and the HMOs used in the invention is shown below.

First, a raw milk material, water according to the need, other components and the like are mixed, preferably homogenized and heat sterilized. The homogenization and the heat sterilization can be conducted by general methods. A lactic acid bacterium starter is added (seeded) to the heat sterilized modified milk solution, and the solution is kept at a certain fermentation temperature and fermented to obtain a fermented product. Through fermentation, curd is formed.

As the lactic acid bacterium starter, for example, lactic acid bacteria which are generally used for yogurt production, such as *Lactobacillus bulgaricus, Lactococcus lactis* and *Streptococcus thermophilus,* can be used. When the pH reaches the target value, the formed curd is crushed by stirring and cooled to 10°C or lower, and a fermented product is thus obtained. By cooling to 10°C or lower, the activity of the lactic acid bacterium can be reduced, and the formation of an acid can be inhibited.

Next, the fermented product obtained by the fermentation step is heat treated, and a fermented product after the heat treatment is thus obtained. By appropriately heating the fermented product, the formation of an acid by the lactic acid bacterium in the fermented product after the heat treatment can be inhibited. As a result, a decrease in pH during the subsequent production step and/or during the storage of the concentrated fermented milk can be inhibited, and as a result, the viability of the Bifidobacterial strain can be improved.

*Bifidobacterium longum* subspecies *infantis,* LNnT, LNT, 2'-FL, 3'-SL and 6'-SL are added to the fermented product after the heating obtained by the heat treatment step. The amount of the *Bifidobacterium longum* subspecies *infantis* added, relative to the fermented product after the heat treatment, is preferably 1×10⁷ to 1×10¹¹ CFU/mL, more preferably 1×10⁸ to 1×10¹⁰ CFU/mL. When the *Bifidobacterium longum* subspecies *infantis* is a dead bacterium, CFU/mL can be replaced with cells/mL.

After adding the *Bifidobacterium longum* subspecies *infantis* and the HMOs to the fermented product after the heat treatment, the mixture is concentrated. The concentration step can be conducted appropriately using a known concentration method. Through the centrifugation method, the whey in the product to be concentrated (the fermented product after the heating containing the Bifidobacterial strain and the prebiotics) is removed, and a concentrated fermented milk which has an increased solid content concentration and which contains the *Bifidobacterium longum* subspecies *infantis* and the HMOs used in the invention is obtained. By taking the obtained fermented milk, the enteric flora can be improved.

### <Production Example 4>

A production method of a formula containing *Bifidobacterium longum* subspecies *infantis* and the HMOs used in the invention is shown below.

In 300 kg of warm water, 10 kg of desalted cow's milk whey protein powder (manufactured by MILEI GmbH), 6 kg of cow's milk casein powder (manufactured by Fonterra Cooperative Group), 48 kg of lactose (manufactured by MILEI GmbH), 920 g of a mineral mixture (manufactured by Tomita Pharmaceutical Co., Ltd), 32 g of a vitamin mixture (manufactured by Tanabe Seiyaku Co., Ltd.), 500 g of lactulose (manufactured by Morinaga Milk Industry Co., Ltd.), 500 g of raffinose (manufactured by Nippon Beet Sugar Manufacturing Co., Ltd.) and 900 g of galactooligosaccharide syrup (manufactured by Yakult Pharmaceutical Industry Co., Ltd.) are dissolved and further heated and dissolved at 90°C for 10 minutes, and after adding 28 kg of modified fats (manufactured by Taiyo Yushi Corp.), the mixture is homogenized. Then, after sterilization and concentration steps, the mixture is spray dried, and about 95 kg of a modified milk is thus prepared. To the modified milk, 100 g of bacterial powder of *Bifidobacterium longum* subspecies *infantis* triturated in starch, LNnT, LNT, 2'-FL, 3'-SL and 6'-SL are added, and about 95 kg of a formula is thus prepared. The obtained formula is dissolved in water, and a modified milk solution having a total solid content concentration as a standard formula concentration of 14% (w/V) is thus obtained. As the bacterial count of the *Bifidobacterium longum* subspecies *infantis* in the obtained formula solution, 2.7×10⁹ CFU/100 ml can be obtained. By administering the formula obtained as described above, the growth of the probiotic in the intestines can be promoted.

By administering the composition, the pharmaceutical composition, the fermented milk or the formula produced in Production Examples 1 to 4 above to a human, *Bifidobacterium longum* subspecies *infantis* grows in the intestines, which leads to the improvement of the enteric flora.

## Claims

1. A composition containing
a human milk oligosaccharide mixture containing
lacto-N-neotetraose,
lacto-N-tetraose,
2'-fucosyllactose,
3'-sialyllactose,
6'-sialyllactose and
*Bifidobacterium longum* subspecies *infantis.*

2. The composition according to claim 1, wherein the *Bifidobacterium longum* subspecies *infantis* is *Bifidobacterium longum* subspecies *infantis* NITE BP-02623.

3. The composition according to claim 1 or 2,
wherein the human milk oligosaccharide mixture contains
the lacto-N-neotetraose in an amount of 2 to 10 mass%,
the lacto-N-tetraose in an amount of 10 to 20 mass%,
the 2'-fucosyllactose in an amount of 55 to 75 mass%,
the 3'-sialyllactose in an amount of 1 to 10 mass% and
the 6'-sialyllactose in an amount of 5 to 15 mass%.

4. The composition according to any one of claims 1 to 3, wherein the human milk oligosaccharide mixture further contains difucosyllactose or 3'-fucosyllactose.

5. The composition according to claim 4,
wherein the human milk oligosaccharide mixture contains the difucosyllactose or the 3'-fucosyllactose in an amount of 1 to 10 mass%.

6. The composition according to any one of claims 1 to 5, further containing lactose.

7. The composition according to any one of claims 1 to 6, wherein the composition is a food or a drink composition or a nutritional composition.

8. The composition according to any one of claims 1 to 6, wherein the composition is a formula.

9. A composition containing
lacto-N-tetraose and
*Bifidobacterium longum* subspecies *infantis.*
